# EUROPEAN PATENT APPLICATION

(11) **EP 0 841 070 A2**
(43) Date of publication of application: **13.05.1998**
(21) Application number: 97119339.6
(22) Date of filing: 05.11.1997
(51) Int. Cl.: A61L 9/12

(54) **Method for producing wicks for containers of vaporizable solutions**

(30) Priority: 12.11.1996 IT BO960576
(71) Applicant: FALP S.r.l., 40060 Passo Segni Baricella (Bologna) (IT)
(72) Inventor: Falchieri, Roberto, 40060 Passo Segni di Baricella, Bologna (IT)
(74) Representative: Modiano, Guido, Dr.-Ing.

(57) **Abstract**

The method for producing wicks meant for containers of vaporizable solutions, such as insecticidal solutions, deodorants and the like, entails mixing with a binder particles of non-cohesive materials chosen among particles of wood, kaolin and coal; blending the resulting mix with a spray of water and by means of a high-speed mixer or turbomixer; extruding the resulting compound so as to obtain pieces which have the dimensions of the wicks; and drying the pieces.

## Description

The present invention relates to a method for producing wicks meant to be part of containers of vaporizable solutions, such as insecticidal solutions, deodorants and the like.

Heaters or electric vaporizers are currently known in which containers of said solutions are placed and which cause the evaporation of said solutions by heating the upper end of a wick which dips into the container of the solution.

The interest shown by consumers for these devices is due to the long life of a charge of the solution, i.e., to the fact that the electric vaporizer can be used for several days before the solution placed in a container is consumed and the empty container must therefore be replaced with a full one.

The wicks or absorbing elements used in this particular field are made of various materials having different degrees of porosity; they are mostly obtained by shaping agglomerates of materials in powder form.

The problem that occurs in the production of wicks is not only to maintain their efficiency for all the required time but also, and most of all, to obtain in a given production cycle wicks which all have constantly the same characteristics.

The aim of the present invention is to solve the described problem and to ensure the production of wicks which are meant to equip containers of vaporizable solutions and which ensure efficiency and constancy of their characteristics.

Within the scope of this aim, an object of the present invention is to provide a method which is simple, easy to manage and economically advantageous.

This aim and this object are achieved, according to the present invention, by the present method for producing wicks meant for containers of vaporizable solutions, such as insecticidal solutions, deodorants and the like, said method being characterized in that it entails mixing with a binder particles of non-cohesive materials chosen among particles of wood, kaolin and coal; blending the resulting mix with sprinkled water and by means of a high-speed mixer or turbomixer; extruding the resulting blend so as to obtain pieces which have the dimensions of the wicks; and drying said pieces.

Water is part of said blend with a weight ratio between 4:10 and 9:10 of said mix.

The water introduced in the high-speed mixer to blend said mix by means of said mixer is atomized.

If said mix is blended by means of the turbomixer, the water is instead introduced in said turbomixer drop by drop or in atomized form.

Said mix of particles of non-cohesive materials and binder is produced in the high-speed mixer or in the turbomixer in which said blend is then formed.

The high-speed mixer and the turbomixer are provided with a cooling system and their internal temperature is controlled so it does not exceed 20 to 25^{o}C in said blend.

The binder is present in said mix with a weight ratio of 0.5:10 to 1.5:10 of particles of non-cohesive materials.

The binder is preferably powdered starch; gum arabic in powder form can also be used as a binder.

Preferably, said mix contains wood, kaolin and coal in powder form and substantially in the same weight.

Further characteristics and advantages will become apparent from the following description of some preferred examples of practical embodiment of the method according to the present invention for producing wicks meant for containers of vaporizable solutions, such as insecticidal solutions, deodorants and the like; said examples are given hereinafter by way of non-limitative illustration.

### EXAMPLE 1

3 kg of kaolin, coal and wood in powder form are taken.

The materials are placed in a turbomixer with 1 kg of powdered starch.

The turbomixer is run at 300 rpm for 7 to 8 minutes so as to produce a mix of the three materials and of the binder.

8.5 liters of water are then fed drop by drop into the turbomixer and the turbomixer is run for another 7 to 8 minutes.

The temperature of the compound being formed inside the turbomixer is kept under control and the water-circulation cooling system of the turbomixer is activated so that the compound that is extracted from it has a temperature of no more than 20 to 25 ^{o}C.

The compound is extruded, obtaining pieces from it which have the dimensions of the wicks meant to equip containers of vaporizable solutions, such as insecticidal solutions, deodorants and the like: the length of said pieces can vary between 20 and 150 mm and their diameter can vary between 5 and 10 mm.

The extruded parts are dried in a dryer for 48 hours if the temperature of said dryer is 30^{o}C or for 24 hours if the temperature of said dryer is 45^{o}C.

It has been observed that all the wicks formed from the blend processed by the turbomixer constantly have the same characteristics.

In particular, wicks with a length of 60 mm and a diameter of 8 mm, used to equip containers which contain 25 ml of pyrethrum solution, are considered; when one of said containers is inserted in an electric vaporizer whose operating temperature is 50^{o}C, the entire solution of the container is uniformly vaporized during a discontinuous activation of the electric vaporizer lasting approximately 45 days.

### EXAMPLE 2

9 kg of wood powder are taken and placed in a high-speed mixer with 1 kg of powdered gum arabic; the mixer is run at 200 rpm for 5 to 6 minutes so as to produce a mix of the two components.

8 liters of water are introduced, nebulizing them, while the mixer runs for another 5 to 6 minutes.

The resulting blend is extruded and cut into pieces which are dried as mentioned, obtaining wicks with a length of 60 mm and a diameter of 8 mm; by using the wicks as in example 1, the solution is vaporized in approximately 40 days of discontinuous operation of the electric vaporizer.

## Claims

1. A method for producing wicks for containers of vaporizable solutions, such as insecticidal solutions, deodorants and the like, said method comprising the steps of mixing with a binder particles of non-cohesive materials chosen among particles of wood, kaolin and coal, blending the resulting mix with sprinkled water and by means of a high-speed mixer or turbomixer, extruding the resulting blend so as to obtain pieces which have the dimensions of the wicks, and drying said pieces.

2. The method according to claim 1, characterized in that in said compound said water is present with a weight ratio between 4:10 and 9:10 of said mix.

3. The method according to claim 1, characterized in that the water is introduced in the high-speed mixer, in nebulized form.

4. The method according to claim 1, characterized in that the water is introduced in said turbomixer, drop by drop or in nebulized form.

5. The method according to claim 1, characterized in that said mix of particles of non-cohesive materials and binder is formed in said high-speed mixer or turbomixer, in which said blend is then produced.

6. The method according to claim 1, characterized in that said high-speed mixer and said turbomixer have a cooling system and in that their internal temperature is controlled so that it does not exceed 20 to 25^{o}C in said blend.

7. The method according to claim 1, characterized in that in said mix the binder is present in a weight ratio of 0.5-1.5 to 10 of said particles of non-cohesive materials.

8. The method according to claim 1, characterized in that said binder is preferably powdered starch.

9. The method according to claim 1, characterized in that wood, kaolin and coal are present in said mix in powder form and substantially in a same weight.
